# FASCICULE DE BREVET EUROPEEN

(11) **EP 0 564 646 B1**
(45) Date de publication et mention de la délivrance du brevet: **19.04.2000**
(21) Numéro de dépôt: 93900220.0
(22) Date de dépôt: 30.10.1992
(51) Int. Cl.: A61K 48/00, C12N 5/10

(54) **CELLULES PIEGEES ET LEUR UTILISATION COMME MEDICAMENT**
GEFALLENE ZELLEN UND IHRE VERWENDUNG IN ARZNEIMITTELN
TRAPPED CELLS AND USE THEREOF AS A DRUG

(30) Priorité: 30.10.1991 FR 9113430
(43) Date de publication de la demande: 13.10.1993
(60) Demande divisionnaire: 99115964.1
(73) Titulaire: UNIVERSITE PIERRE ET MARIE CURIE PARIS VI, 75252 Paris Cédex 05 (FR)
(72) Inventeur: KLATZMANN, David, F-75013 Paris (FR)
(74) Mandataire: Gutmann, Ernest
(86) Numéro de dépôt international: FR9201016
(87) Numéro de publication internationale: WO9308843

(56) Documents cités:
- WO-A-91/12849
- CELL STRUCTURE AND FUNCTION vol. 12, 1987, JAPAN SOCIETY FOR CELL BIOLOGY pages 205 - 217 REIKO SASADA ET AL. 'THE ESTABLISHMENT OF IL-2 PRODUCING CELLS BY GENETIC ENGINEERING'
- SCIENCE. vol. 208, 30 Mai 1980, LANCASTER, PA US pages 1033 - 1035 KAREN E. MERCOLA ET AL. 'INSERTION OF A NEW GENE OF VIRAL ORIGIN INTO BONE MARROW CELLS OF MICE'
- Blood, vol.89, 1997, pp.4636-4645
- Science, vol.276, 1997, pp.1719-1724

## Description

L'invention est relative à des cellules piégées à l'égard d'antigènes provoquant de dysfonctionnements du système immunitaire, plus particulièrement de ceux qui sont impliqués dans des dysfonctionnements naturels (maladies auto-immunes) ou provoqués (greffes) du système immunitaire.

L'invention est plus particulièrement relative à la constitution de médicaments pour la prévention de maladies du type "Graft Versus Host Diseases" (GVHD) (maladies dues à la réaction d'un greffon contre l'hôte) ou pour le traitement de pathologies auto-immunes, plus particulièrement en association avec lesdites cellules piégées.

Dans ce qui suit l'expression "antigène" vise toutes substances ou tous produits contre lesquels une tolérance de l'hôte est recherchée, alors qu'elles tendraient de façon naturelle à induire une réaction de défense ou de rejet de la part du système immunitaire de l'hôte.

L'invention a pour but de remédier à ces inconvénients, plus particulièrement de proposer une approche thérapeutique différente, fondée sur la présence, assurée au préalable chez l'hôte, de cellules lymphoïdes, notamment lymphocytes T piégées à l'égard d'une activation par les antigènes pour lesquels une tolérance est recherchée.

L'invention concerne donc plus particulièrement une population de cellules hématopoïétiques, piegées à l'égard d'une activation par des antigènes normalement étrangers à l'hôte, mais à l'égard duquel une tolérance est recherchée, caractérisée en ce que ces cellules contiennent une séquence génétique dont le produit d'expression peut, lorsque sa production est accrue pour en obtenir une quantité suffisante au sein de ces cellules piégées, réagir in situ avec une substance pharmaceutiquement active pour induire la destruction de ces cellules, et des moyens spécifiquement inductibles par un signal d'activation fourni à ces cellules par l'antigène, pour déclencher la susdite production accrue.

L'administration d'une telle substance pharmaceutiquement active à un hôte porteur d'une telle population de cellules piégées, peut permettre la protection efficace de l'hôte contre une activation par des antigènes des cellules lymphoïdes spécifiques appropriées du système immunitaire, notamment de ses lymphocytes T, dès lors que ces cellules lymphoïdes doivent être détruites avant même qu'elles aient commencées à se diviser. En raison de la destruction des cellules piégées commandée par l'antigène lui-même, leur division sera bloquée.

D'une façon générale les techniques mises en oeuvre à ce jour pour faire face aux affections résultant d'une intolérance du système immunitaire, tendent, soit à provoquer une immunodépression généralisée chez l'hôte, soit à modifier certaines catégories des cellules affectées par ce type d'antigène, de façon à leur conférer la capacité de produire de façon constitutive certaines substances supposées inhiber le développement de tels antigènes.

L'invention concerne plus particulièrement une population de cellules de la moelle osseuse, notamment de cellules immatures utilisables pour des greffes de moelle osseuse, ces cellules étant "piégées" dans les conditions sus-indiquées.

L'invention concerne donc également l'utilisation pour la production de médicaments actifs contre des maladies du type GVHD d'une molécule pharmaceutiquement active capable, lorsqu'elle se trouve associée in vivo avec une population de cellules hématopoïétiques, piégées comme cela a été décrit plus haut et réintroduites dans l'organisme de l'hôte, notamment à la suite d'une greffe de moelle osseuse, d'interagir avec le produit d'expression de la séquence génétique contenue dans ces cellules piégées causer leur destruction, dès lors qu'elles seraient stimulées par les antigènes contre lesquels une tolérance est recherchée.

Dans une forme de mise en oeuvre préférée de l'invention, la population de cellules piégées est caractérisée en ce que les moyens permettant le déclenchement spécifique de la production accrue du produit d'expression et la séquence génétique susdite sont contenus dans une séquence d'ADN recombinant contenu dans ces cellules. Avantageusement, les moyens spécifiquement inductibles par un signal résultant de l'activation de ces cellules par ces antigènes, consistent en des séquences régulatrices contrôlant normalement l'expression d'une cytokine, notamment lymphokine, par exemple de l'interleukine 2, ou l'expression de récepteurs correspondants de cette cytokine, notamment le récepteur de l'interleukine 2, et la séquence génétique susdite est alors placée sous le contrôle de ce promoteur et code pour un produit d'expression capable, lorsque sa production est accrue sous l'effet de l'activation des susdites séquences régulatrices, de réagir avec une substance pharmaceutiquement active, pour former un produit de réaction capable d'induire directement ou indirectement la destruction des cellules en cours d'activation.

Avantageusement, la séquence régulatrice mise en oeuvre est constituée par un promoteur normalement associé à l'interleukine 2 ou de préférence, à un récepteur de l'interleukine 2. En particulier, on aura recours au promoteur de la chaîne α du récepteur de l'interleukine 2 (séquence décrite dans Cell Avril, 1987). Ce promoteur loraqu'il est activé, conduit à une expression amplifiée de la séquence placée sous son contrôle (notamment dans des cellules JURKAT), qui est de l'ordre de 30 fois celle qui est mesurée lorsqu'il n'est pas activé.

Avantageusement la substance pharmaceutique utilisée induit directement ou indirectement l'interruption in situ de la réplication d'ADNs au sein des cellules en cause. De façon encore particulièrement préférée, l'invention fait également application de la technique dite "d'oblitération de thymidine kinase" déjà décrite par Borelli et al (1988) dans des cellules l'exprimant déjà de façon constitutive et dans un champs d'application différent. La population de cellules piégées conformes à un mode de mise en oeuvre préféré de l'invention est alors caractérisée en ce que le produit d'expression codée par ladite séquence génétique placée sous le contrôle dudit promoteur, est une molécule qui, telle la thymidine kinase du virus de l'herpès simplex 1 (HSV1-TK), est apte, lorsqu'elle est présente en une concentration suffisante dans les cellules en cause, à phosphoryler des analogues de nucléosides, tels que l'acyclovir (9-[(2-hydroxyethoxy)méthyl)guanine) ou le gancyclovir (9-[1,3-10hydroxy-2-propoxyméthyl]guanine), en des molécules monophosphatées, elles-mêmes convertibles par des enzymes cellulaires en nucléotides triphosphatés incorporables dans des acides nucléiques en cours d'élongation sous l'effet des polymérases au sein desdites cellules, avec pour effet l'interruption d'élongation de chaînes et la mort cellulaire qui s'ensuit.

Il apparaîtra immédiatement que la "substance pharmaceutique active" utilisable dans un schéma thérapeutique mettant en oeuvre l'une des différentes populations de cellules piégées telles que définies ci-dessus à titre préférentiel doit, dans chaque cas, présenter les propriétés qui lui permettront de réagir avec le produit d'expression correspondant, dans les conditions qui ont été définies.

L'invention met en fait à profit deux phénomènes distincts :
1) l'expression d'un gène toxique placé sous le contrôle d'un promoteur de cytokine ou de récepteur de cytokine dans des cellules piégées conformes à l'invention est tout au plus très faible, tant qu'elles ne sont pas activées par l'antigène en cause ;
2) l'induction de l'expression de la séquence génétique toxique peut être rendue suffisamment rapide dans celle des cellules piégées qui serait activée, pour déclencher leur destruction avant même qu'interviennent les premières divisions cellulaires de ces cellules. Par conséquent l'activation du système immunitaire de l'hôte, lorsqu'il est stimulé par un antigène contre lequel une tolérance est en fait recherchée, se trouve bloquée dans sa phase initiale.

Pour construire des cellules piégées conformes à l'invention pour l'homme -ou le cas échéant par l'animal- on peut avoir recours à toute technique adéquate, notamment par infection in vitro des cellules correspondantes par une pseudo-particule virale de type Moloney amphotrope, qui infecte donc aussi des cellules humaines. Ces particules virales sont produites par une lignée cellulaire dite de "packaging" que l'on aura construite au préalable. Une lignée de packaging est capable de fabriquer tous les éléments structuraux constituant une particule virale, mais est incapable d'introduire dans des particules virales en voies de maturation les ARNs viraux produits par cette lignée cellulaire. C'est pourquoi, ces lignées dites de packaging fabriquent en permanence des particules virales vides. L'introduction d'une construction génétique appropriée, qui contient l'ADN recombinant tel qu'il a été défini plus haut, permet à ces lignées de packaging d'être introduites dans les particules virales vides réalisant ainsi des pseudo-particules virales. Ces pseudo-particules virales sont capables d'infecter différentes cellules cibles, cellules cibles qui varient selon la lignée de packaging qu'on a utilisée au départ. Par exemple, si cette lignée de packaging est dérivée d'un virus dit de Moloney amphotrope, les particules virales produites infectent parfaitement les cellules hématopoïétiques humaines.

Ainsi les cellules hématopoïétiques à piéger sont-elles mises en présence d'un surnageant de culture provenant de la lignée de packaging qui produit les pseudo-particules virales amphotropes. Les cellules à piéger sont par exemple co-cultivées directement avec les cellules de packaging. Pendant cette étape de co-culture ou de culture, les particules amphotropes présentes dans la suspension infectent les cellules à piéger et introduisent donc dans celles-ci les éléments génétiques qu'elles contiennent.

Pour la production de ces cellules piégées mettant plus particulièrement en oeuvre un promoteur de lymphokine ou de récepteur de lymphokine, on aura avantageusement recours aux vecteurs rétroviraux murins amphotropes et aux lignées cellulaires de packaging (respectivement telles qu'elles ont été revues par Weatherall, 1991 et Friedman, 1989). Les techniques pour la production de lignées de cellules transformées par un tel vecteur rétroviral (voir par exemple Danos et al, 1988 et Markowitz et al, 1989), peuvent être transposées à la production de cellules piégées hématopoïétiques conformes à l'invention. De même les techniques de transfert génétique mettant en oeuvre de tels systèmes (Kasid et al, 1990) peuvent-elles être appliquées au transfert, chez l'homme, des cellules piégées telles qu'elles ont été définies plus haut.

Une population intéressante de cellules piégées conformes à l'invention est dérivée de cellules de moelle osseuse immatures, provenant d'un prélèvement de moelle osseuse, notamment dépourvues des marqueurs de surface caractérisant des cellules hématopoïétiques matures, et préalablement transformées en "cellules piégées", dans les conditions sus-indiquées. Elles sont particulièrement adaptées à la mise en oeuvre de l'un des protocoles thérapeutiques dont les étapes sont brièvement rappelées ci-après.

Les étapes essentielles de ce protocole sont alors les suivantes :
- prélèvement de moelle osseuse chez le patient, et traitement de cette moelle osseuse selon les techniques de l'art, de façon à purifier les cellules souches immatures ;
- incorporation dans ces cellules immatures du susdit ADN recombinant contenant le promoteur activable par l'antigène et, placé sous le contrôle de ce promoteur, la séquence génétique codant par exemple pour HSVI-TK dont le produit d'expression peut interagir avec la substance pharmaceutiquement active administrée ultérieurement, en l'occurrence des analogues appropriés de nucléosides du type acyclovir ou gancyclovir, pour bloquer l'activation par l'antigène des cellules affectées par cet antigène ;
- Les cellules de la moelle osseuse obtenue, qui contient alors l'ADN recombinant défini plus haut, sont soit réinjectées en totalité chez le patient, soit soumises à une étape de culture supplémentaire et de sélection pour obtenir une quantité de cellules ayant effectivement été infectées par les particules amphotropes et donc contenant et exprimant l'information génétique contenue dans les particules pseudo-virales. Les cellules de moelle osseuse manipulées sont réinjectées par voie intraveineuse au patient qui aura été préalablement conditionné, par exemple par traitement avec de fortes concentrations d'antiviraux appropriés, utilisés à des doses fortes même si celles-ci sont relativement toxiques pour la moelle osseuse puisque le patient recevra par la suite de nouvelles cellules de moelle osseuse. Ce conditionnement préalable du patient peut même impliquer une destruction in vivo de son système immunitaire initial, notamment par irradiation. Dans cette dernière hypothèse, les cellules piégées réinjectées chez le patient -ou les cellules piégées provenant d'une moelle osseuse issue d'un donneur différent, traitées comme indiquées ci-dessus et injectées au patient- sont alors aptes à se renouveler constamment chez l'homme et à donner naissance aux différentes catégories de cellules hématopoïétiques qui, alors, portent toutes la séquence recombinante ;
- administration de la substance pharmaceutiquement active au patient pendant le temps et au moment requis, notamment pour entraîner la destruction sélective des cellules piégées, lorsque celles-ci sont activées par l'antigène, suite à l'interaction de la substance pharmaceutiquement active avec le produit d'expression, notamment HSVI-TK alors produit in situ en concentration suffisante dans ces cellules piégées sous l'effet de l'activation du promoteur, notamment celui normalement associé à une interleukine 2 ou à un récepteur d'interleukine 2.

Naturellement le choix du moment de l'administration, notamment par exemple par rapport à une greffe d'organe ou de moelle osseuse étrangère chez l'hôte, ainsi que les modalités de l'administration de la substance pharmaceutiquement active, notamment l'acyclovir ou le gancyclovir, doivent être mises au point par la clinicien. Elles comportent, par exemple, un traitement continu dans un premier temps, itératif dans un second temps.

On appréciera que l'invention présente plusieurs avantages, notamment quant à la possibilité qu'a le clinicien de maîtriser à la fois :
- le déclenchement, au moment de son choix, de l'interaction du produit d'expression de la séquence codante avec la substance pharmaceutiquement active administrée dans celles des cellules dont le promoteur serait activé par l'antigène ou les antigènes ;
- l'interruption au moment choisi de l'interaction sus-mentionnée, grâce à l'arrêt de l'administration du médicament ;
- si nécessaire, la réinstitution du traitement, également au moment de son choix.

Des exemples quant aux conditions de mise en oeuvre de l'invention sont encore indiquées ci-après, naturellement à titre non limitatif :

### A. Utilisation pour le traitement préventif de la réaction du greffon contre l'hôte

Dans le cadre d'une transplantation de moelle, en particulier lorsqu'il s'agit de transplantation réalisée en l'absence d'une compatibilité totale entre le donneur et le receveur, la moelle prélevée sur le donneur serait traitée comme indiqué précédemment. La construction génétique présente dans les pseudo-particules virales présenterait le gène suicide sous le contrôle du promoteur du gène codant pour l'interleukine 2 ou du promoteur du gène codant pour le récepteur à l'interleukine 2.

Le receveur de la moelle serait irradié avant réinjection de la moelle. Il serait ensuite traité de façon préventive par l'acyclovir ou toute drogue analogue, pendant toute la durée de la reconstitution immunologique. Le traitement pourrait être effectué de façon continue ou discontinue. Ainsi si certaines cellules provenant de la moelle greffée sont sensibilisées par les antigènes du receveur (réaction de GVH), elles seront alors éliminées grâce au traitement. Une fois la reconstitution immunologique obtenue le traitement pourrait être arrêté car en principe toutes les cellules potentiellement dangereuses auront été éliminées. Néanmoins, au cours de l'évolution ultérieure de la greffe, toute possible réactivation d'une réaction du greffon contre l'hôte qui serait diagnostiquée comme telle pourrait être traitée par les analogues nucléosidiques avec le même effet bénéfique.

### B. Utilisation pour le traitement des pathologies auto-immunes

Dans le cas des pathologies auto-immunes, la situation est très proche de celle décrite précédemment, à ceci près que la moelle osseuse provient du même individu. Dans ce type d'intervention thérapeutique, la moelle osseuse est prélevée directement chez le malade et après manipulation identique à celle décrite précédemment, elle est réinjectée au malade préalablement irradié. La reconstitution est alors obtenue rapidement et au cours de tout événement clinique d'auto-immunité, le patient est traité par les analogues nucléosidiques. Par exemple pour des patients souffrant de maladies rhumatismales de caractère auto-immun, la réapparition de signes cliniques de type rhumatismal pourrait entraîner la remise en place du traitement par les analogues nucléosidiques. En principe, il est tout à fait imaginable que ce traitement aboutisse à l'élimination définitive de toutes les cellules potentiellement auto-réactives. Néanmoins si tel n'était pas le cas à chaque fois que les signes cliniques réapparaîtraient, le traitement serait réinstitué.

L'invention, ne saurait être limitée aux modes de réalisation qui ont été plus particulièrement illustrés dans les exemples, en particulier ceux mettant en oeuvre le couple d'éléments respectivement formés par la séquence génétique codant pour la HSV1-TK et la "substance pharmaceutiquement active" constituée par l'acyclovir ou un analogue de ce nucléoside modifié.

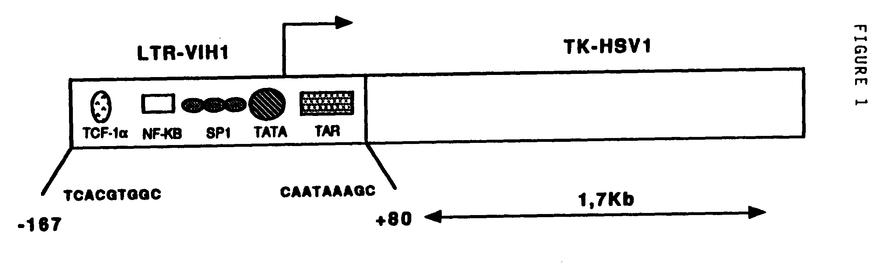

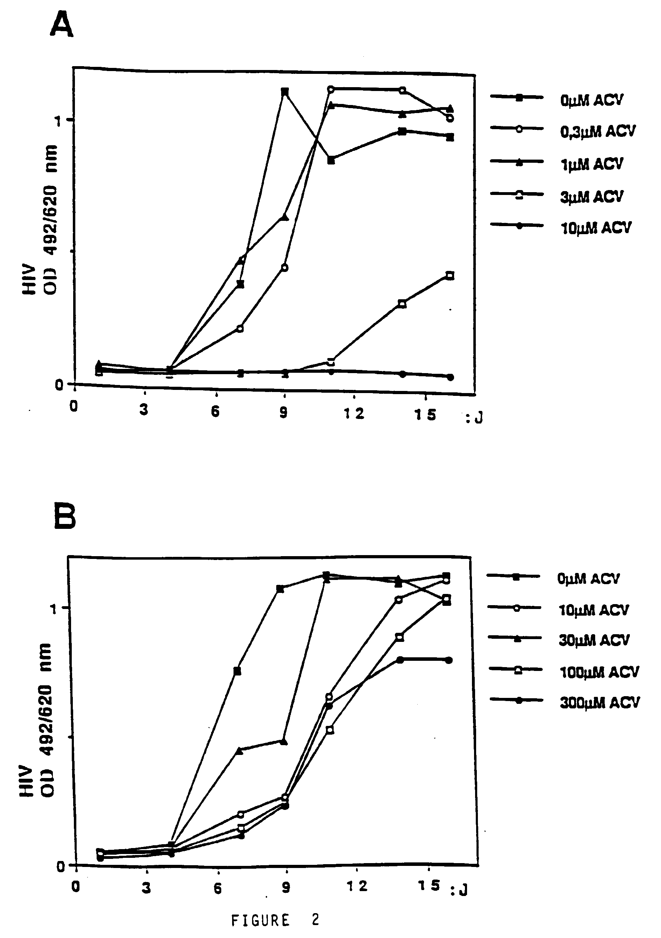

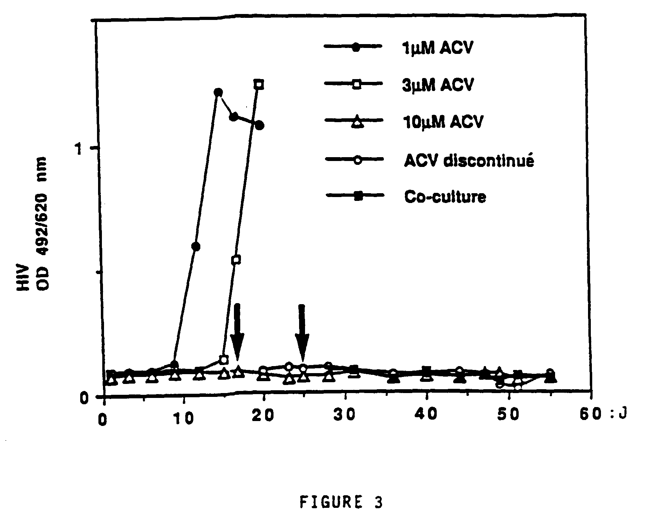

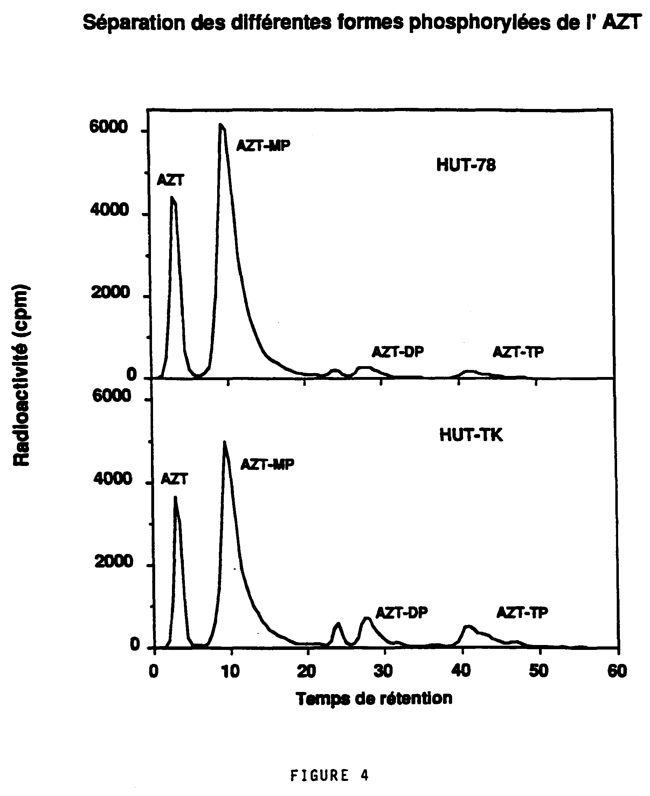

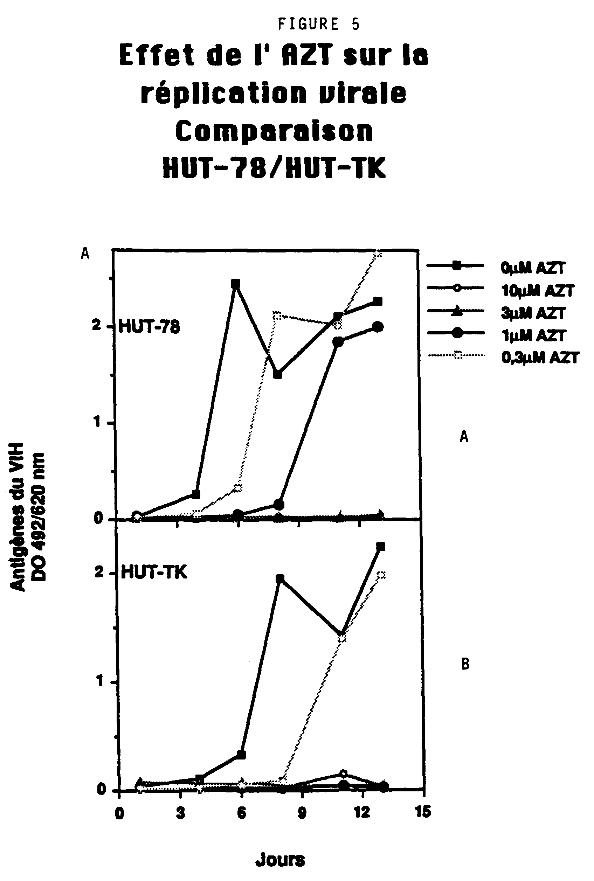

## Revendications

1. Population de cellules caractérisée en ce qu'elle comprend des cellules hématopoïétiques piégées à l'égard de l'activation par un antigène contre lequel une tolérance du système immunitaire est recherchée, et en ce que ces cellules hématopoïétiques piégées contiennent un acide nucléique recombinant comprenant :
- une séquence de régulation exprimée dans lesdites cellules hématopoïétiques lorsqu'elles sont activées en réponse audit antigène, et, placée sous le contrôle de cette région,
- une séquence codant pour une molécule apte à phosphoryler un analogue de nucléoside tel que l'acyclovir ou le gancyclovir en une molécule monophosphatée, elle-même convertible par des enzymes cellulaires en nucléotide triphosphaté incorporable dans des acides nucléiques en cours d'élongation sous l'effet des polymérases au sein desdites cellules, avec pour effet l'interruption d'élongation de chaînes.

2. Population de cellules selon la revendication 1 caractérisée en ce que la molécule apte à phosphoryler des analogues de nucléosides tels que l'acyclovir ou le gancyclovir est la thymidine kinase du virus de l'herpès simplex de type 1 (HSV1-TK).

3. Population de cellules selon la revendication 1 ou 2 caractérisée en ce que la séquence de régulation est une séquence de régulation contrôlant normalement l'expression d'une cytokine ou de l'un des récepteurs correspondants.

4. Population de cellules selon la revendication 3 caractérisée en ce que la séquence de régulation est une séquence de régulation contrôlant normalement l'expression d'une lymphokine, par exemple de l'interleukine-2.

5. Population de cellules selon l'une quelconque des revendications 1 à 4 caractérisée en ce que les cellules hématopoïétiques sont des lymphocytes.

6. Population de cellules selon la revendication 5 caractérisée en ce que les cellules hématopoïétiques sont des lymphocytes T.

7. Population de cellules selon l'une quelconque des revendications 1 à 4 caractérisée en ce que les cellules hématopoïétiques sont des cellules dérivées de cellules souches de moelle osseuse.

8. Utilisation, pour la préparation d'une composition pharmaceutique destinée à induire une tolérance contre des antigènes qui tendraient, normalement, à induire une réaction de défense ou de rejet par le système immunitaire d'un hôte:
- d'une population de cellules selon l'une des revendications 1 à 7, et,
- d'un analogue de nucléoside tel que défini dans la revendication 1.

9. Utilisation, pour la préparation d'une composition pharmaceutique destinée au traitement de pathologies autoimmunes:
- d'une population de cellules selon l'une des revendications 1 à 7, et,
- d'un analogue de nucléoside tel que défini dans la revendication 1.

10. Utilisation, pour la préparation d'une composition pharmaceutique destinée au traitement, notamment préventif, de la réaction du greffon contre l'hôte:
- d'une population de cellules selon l'une des revendications 1 à 7, et,
- d'un analogue de nucléoside tel que défini dans la revendication 1.

11. Utilisation selon l'une des revendications 8 à 10 caractérisée en ce que l'analogue de nucléoside est choisi parmi le gancyclovir et l'acyclovir.

12. Utilisation selon l'une des revendications 8 à 11 caractérisée en ce que l'analogue de nucléoside est administré postérieurement à la population de cellules.

13. Association à des fins thérapeutiques :
- d'une polulation de cellules selon l'une des revendications 1 à 7, et,
- d'un analogue de nucléoside tel que défini dans la revendication 1.

## Claims

1. Population of cells, characterized in that it comprises hematopoietic cells trapped against activation by an antigen in resped to which a tolerance of the immune system is sought and in that these trapped hematopoietic cells contain a recombinant nucleic acid comprising :
- a regulatory sequence expressed in said hematopoietic cells when they are activated in response to said antigen and placed under the control of this region,
- a sequence coding for a molecule able to phosphorylate a nucleoside analogue such as acyclovir or gancyclovir to form a monophosphated molecule, which can itself be converted by cellular enzymes to a nucleoside triphosphate which can be incorporated into nucleic adds during elongation catalyzed by the polymerases within said cells and thus cause the interruption of chain elongation.

2. Population of cells according to Claim 1, characterized in that the molecule able to phosphorylate nucleoside analogues such as acyclovir or gancyclovir is the thymidine kinase of the herpès simplex 1 virus (HSV1-TK).

3. Population of cells according to Claim 1 or 2, characterized in that the regulatory sequence is a regulatory sequence which normally regulates the expression of a cytokine or one of the corresponding receptors.

4. Population of cells according to Claim 3, characterized in that the regulatory sequence is a regulatory sequence which normally regulates the expression of a lymphokine, for example interleukin-2.

5. Population of cells according to any one of the Claims 1 to 4, characterized in that the hematopoietic cells are lymphocytes.

6. Population of cells according to Claim 5, characterized in that the hematopoietic cells are T lymphocytes.

7. Population of cells according to any one of the Claims 1 to 4, characterized in that the hematopoietic cells are cells derived from bone marrow stem cells.

8. Use for the preparation of a pharmaceutical composition designed to induce tolerance against antigens which normally would tend to induce a reaction of defence or rejection by a host immune system of :
- a population of cells according to one of the Claims 1 to 7, and
- a nucleoside analogue such as defined in Claim 1.

9. Use for the preparation of a pharmaceutical composition designed for the treatment of autoimmune diseases of :
- a population of cells according to one of the Claims 1 to 7, and
- a nucleoside analogue such as defined in Claim 1.

10. Use for the preparation of a pharmaceutical composition designed for the treatment, in particular preventive treatment, of a graft-versus-host reaction of :
- a population of cells according to one of the Claims 1 to 7, and
- a nucleoside analogue such as defined in Claim 1.

11. Use according to one of the Claims 8 to 10, characterized in that the nucleoside analogue is either gancyclovir or acyclovir.

12. Use according to one of the Claims 8 to 11, characterized in that the nucleoside analogue is subsequently administered to the population of cells.

13. Combination for therapeutic purposes of :
- a population of cells according to one of the Claims 1 to 7, and
- a nucleoside analogue such as defined in Claim 1.

## Patentansprüche

1. Population von Zellen, dadurch gekennzeichnet, daß sie hämatopoetische Zellen umfaßt, die sich in Hinsicht auf die Aktivierung durch ein Antigen, gegen welches eine Toleranz des Immunsystems angestrebt wird, in einer Falle befinden, und daß diese in einer Falle befindlichen hämatopoetischen Zellen eine rekombinierte Nukleinsäure enthalten, umfassend:
- eine Regulationssequenz, die in den hämatopoetischen Zellen, wenn sie in Reaktion auf das genannte Antigen aktiviert werden, exprimiert wird, und unter die Kontrolle dieses Bereichs gestellt
- eine Sequenz, die ein Molekül kodiert, das in der Lage ist, ein Nukleosidanalog, wie Acyclovir oder Gancyclovir, zu einem monophosphatierten Molekül zu phosphorylieren, welches selbst durch zelluläre Enzyme in Nukleotidtriphosphat, das in Nukleinsäuren im Verlauf der Elongation unter der Wirkung der Polymerasen innerhalb dieser Zellen eingebaut werden kann, was die Unterbrechung der Kettenelongation zur Folge hat, umgewandelt werden kann.

2. Population von Zellen nach Anspruch 1, dadurch gekennzeichnet, daß das Molekül, das in der Lage ist, Nukleosidanaloga, wie Acyclovir oder Gancyclovir, zu phosphorylieren, die Thymidinkinase des Herpes simplex-Virus Typ 1 (HSV1-TK) ist.

3. Population von Zellen nach Anspruch 1 oder 2, dadurch gekennzeichnet, daß die Regulationssequenz eine Regulationssequenz ist, die normalerweise die Expression eines Cytokins oder eines der entsprechenden Rezeptoren kontrolliert.

4. Population von Zellen nach Anspruch 3, dadurch gekennzeichnet, daß die Regulationssequenz eine Regulationssequenz ist, die normalerweise die Expression eines Lymphokins, beispielsweise von Interleukin-2, kontrolliert.

5. Population von Zellen nach einem der Ansprüche 1 bis 4, dadurch gekennzeichnet, daß die hämatopoetischen Zellen Lymphozyten sind.

6. Population von Zellen nach Anspruch 5, dadurch gekennzeichnet, daß die hämatopoetischen Zellen T-Lymphozyten sind.

7. Population von Zellen nach einem der Ansprüche 1 bis 4, dadurch gekennzeichnet, daß die hämatopoetischen Zellen Zellen sind, die von Zellen von Knochenmark-Ursprung abgeleitet sind.

8. Verwendung von:
- einer Population von Zellen nach einem der Ansprüche 1 bis 7 und
- einem Nukleosidanalog, wie in Anspruch 1 definiert, für die Herstellung einer pharmazeutischen Zusammensetzung, die dazu bestimmt ist, eine Toleranz gegen Antigene zu induzieren, die normalerweise darauf abzielen, eine Abwehr- oder Abstoßungsreaktion durch das Immunsystem eines Wirts zu induzieren.

9. Verwendung von:
- einer Population von Zellen nach einem der Ansprüche 1 bis 7 und
- einem Nukleosidanalog, wie in Anspruch 1 definiert, für die Herstellung einer pharmazeutischen Zusammensetzung, die für die Behandlung von Autoimmunerkrankungen bestimmt ist.

10. Verwendung von:
- einer Population von Zellen nach einem der Ansprüche 1 bis 7 und
- einem Nukleosidanalog, wie in Anspruch 1 definiert, für die Herstellung einer pharmazeutischen Zusammensetzung, die für die Behandlung, insbesondere präventive Behandlung, der Reaktion des Transplantats gegen den Wirt (Graft-versus-Host-Reaktion) bestimmt ist.

11. Verwendung nach einem der Ansprüche 8 bis 10, dadurch gekennzeichnet, daß das Nukleosidanalog unter Gancyclovir und Acyclovir ausgewählt wird.

12. Verwendung nach einem der Ansprüche 8 bis 11, dadurch gekennzeichnet, daß das Nukleosidanalog später als die Population von Zellen verabreicht wird.

13. Zusammenstellung zu therapeutischen Zwecken:
- von einer Population von Zellen nach einem der Ansprüche 1 bis 7 und
- einem Nukleosidanalog, wie in Anspruch 1 definiert.
